# EUROPEAN PATENT APPLICATION

(11) **EP 3 605 090 A1**
(43) Date of publication of application: **05.02.2020**
(21) Application number: 19192078.4
(22) Date of filing: 23.05.2013
(51) Int. Cl.: G01N 33/53, G01N 33/58, C07K 16/08

(54) **SELECTION METHOD FOR THERAPEUTIC AGENTS**

(30) Priority: 23.05.2012 US 201261650964 P
(62) Divisional of application: 13794597.8
(71) Applicant: F. Hoffmann-La Roche AG, 4070 Basel (CH)
(72) Inventor: Kelley, Robert F., South San Francisco, California 94080 (US); Theil, Frank-Peter, South San Francisco, California 94080 (US); Bernstein, Lisa, South San Francisco, California 94080 (US); Hotzel, Isidro, South San Francisco, California 94080 (US)
(74) Representative: Buser, Andres

(57) **Abstract**

The invention relates methods for detecting off-target binding of therapeutic candidates. This assay can be used, inter alia, during antibody lead generation or optimization to increase the probability of obtaining a suitable drug.

## Description

### CROSS REFERENCE TO A RELATED APPLICATION

This application claims benefit from United States Provisional Application No. 61/650,964, filed on May 23, 2012, which is incorporated by reference herein in its entirety.

### TECHNICAL FIELD

This invention relates to selection methods for identifying therapeutic agents that have an increased risk of having fast clearance in humans or humans and cynomolgus monkeys, and methods of reducing the clearance of such therapeutic agent.

### BACKGROUND

Human or humanized monoclonal antibodies have been broadly successful as therapeutic agents for human disease. Over 30 antibodies have received FDA-approval for treatment of a variety of disorders with several hundred more currently in clinical development.¹ In addition to the exquisite selectivity and high potency that can be achieved with an antibody therapeutic, the success of antibodies as drugs has greatly benefited from their typically long circulating half-life. A slow clearance from blood enables desired drug concentrations to be realized with infrequent dosing. Antibody drugs are usually administered via intravenous infusion or subcutaneous injection and less frequent administration improves patient compliance and hence clinical benefit.

Antibodies can be eliminated from systemic circulation by several mechanisms - intracellular catabolism following fluid phase endocytosis (non-specific clearance), antigen-mediated clearance⁴, and in some cases due to formation of anti-therapeutic antibodies (ATA). Antigen-mediated clearance is generally observed when antibodies bind to cell-bound antigen, is most prominent at low concentrations of antibody, and can usually be saturated by increasing the antibody dose. ATA have been observed to appear 4-7 days after dosing, involve formation of immune complexes that can be rapidly cleared, and can sometimes be easily detected from the atypical shape of the antibody's plasma concentration-time profile.

Antibody elimination is slowed through an FcRn-dependent recycling mechanism . As an antibody traverses the endocytic pathway, it can bind to FcRn at pH < 6. Binding to FcRn protects IgG from catabolism and promotes return to the apical cell surface where it can be rapidly released at the pH (>7) of blood. These features result in half-lifes of 6-32 days for human or humanized antibodies in humans (Keizer, R.J., Huitema, A.D.R., Schellens, J.H.M., Beijnen, J.H. Clinical Pharmacokinetics of Therapeutic Monoclonal Antibodies. Clinical Pharmacokinetics 49, 493-507 (2010)). On the basis of pharmacokinetic data with 12 IgG1 antibodies, a mean value for clearance in humans of 3.9±1.2 (S.D.) mL/kg/day with range of ∼2-6 mL/kg/day was determined (Deng, R. et al. Projecting human pharmacokinetics of therapeutic antibodies from nonclinical data: What have we learned? mAbs 3, 61-66 (2011)). Similarly, studies of these antibodies in cynomolgus monkeys yielded a mean clearance of 6.5±2.9 (S.D.) mL/kg/day. Engineering of the antibody Fc region to increase binding to FcRn at pH 6.0 can increase half-life of potential therapeutic antibodies in cynomolgus monkeys and in mice (Dall'Acqua, W.F., Kiener, P.A. & Wu, H. Properties of human IgG1s engineered for enhanced binding to the neonatal Fc receptor (FcRn). The Journal of biological chemistry 281, 23514-23524 (2006); Hinton, P.R. et al. An Engineered Human IgG1 Antibody with Longer Serum Half-Life. J Immunol 176, 346-356 (2006); Yeung, Y.A. et al. Engineering human IgG1 affinity to human neonatal Fc receptor: impact of affinity improvement on pharmacokinetics in primates. J Immunol 182, 7663-7671 (2009); Zalevsky, J. et al. Enhanced antibody half-life improves in vivo activity. Nature biotechnology 28, 157-159 (2010)).

Preclinical testing of a potential therapeutic antibody in a relevant non-human species is necessary to gain an understanding of the expected dosing regimen in humans, and to assess potential toxicities. Given the high target antigen sequence homology between human and non-human primates (NHP), and similar binding affinities for the recycling FcRn receptor (Dall'Acqua, Kiener & Wu, *supra*), cynomolgus monkey is the preferred species for preclinical pharmacokinetic (PK) and toxicology studies. Previously we have shown that the non-specific clearance of therapeutic IgG1 antibodies determined in humans is commonly about half that measured in cynomolgus monkeys (Deng, R. *et al., supra*)⁸.

One potential mechanism for faster than expected clearance is off-target binding¹³⁻¹⁶. Although highly specific off-target binding can sometimes be identified and eliminated,¹⁴ most often off-target binding is of unknown origin and difficult to saturate with an increase in dose. *In vitro* systems to evaluate and predict the *in vivo* absorption, distribution, metabolism, and elimination mechanisms or the *in vivo* pharmacokinetic behavior of antibodies are not yet established. We sought to develop *in vitro* assays of non-specific binding that would be useful for identifying antibodies likely to show fast clearance *in vivo.*

### BRIEF DESCRIPTION OF THE DRAWINGS

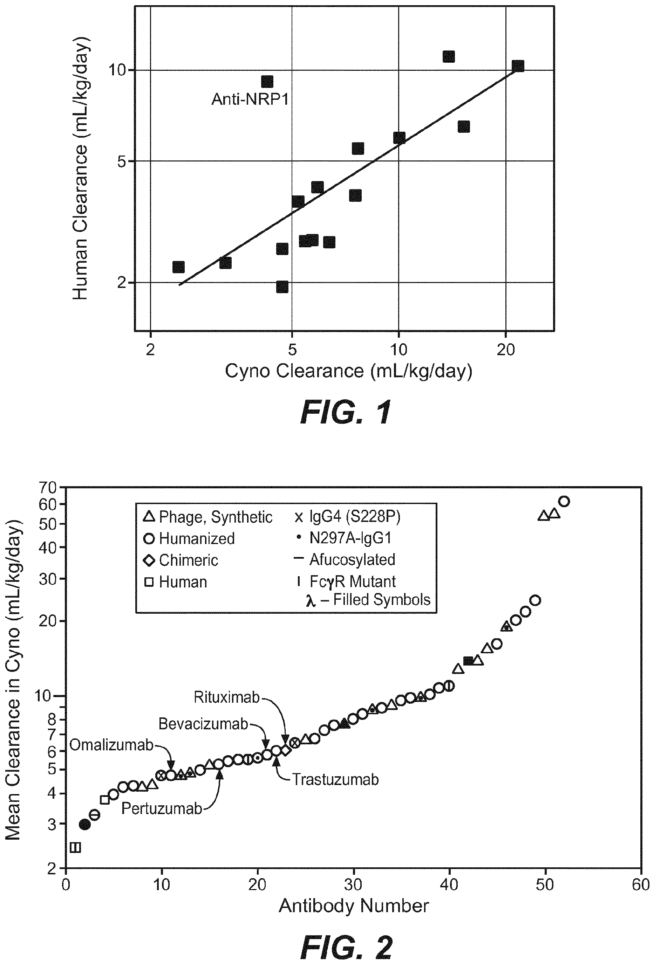

Figure 1 is a graph that shows the correlation of antibody clearance values measured in humans and cynomolgus monkeys (ρ = 0.74, n=16). For our analyses, antibody doses were chosen that were believed to saturate any target-dependent clearance. The solid line is a linear regression fit of the logarithm of human clearance to the logarithm of cynomolgus monkey clearance. For most antibodies shown, human clearance is about 2-fold slower than the corresponding cynomolgus monkey clearance. By contrast, for anti-NRPl the clearance in human (9.2 mL/kg/day) is ∼2-fold *faster* than the clearance in cynomolgus monkey (4.3 mL/kg/day).
Figure 2 is a graph showing the clearance values of antibodies (n=52) in cynomolgus monkeys. Geometric mean values are shown from dose groups where contribution of any specific clearance to the reported clearance was assumed to be negligible. Except for 4 studies, mean values are from 3 or more animals. Humanized (circle), human (square), synthetic human phage derived (triangle), and chimeric (diamond) antibodies are shown. Filled symbols indicate antibodies with a lambda light chain, all others have a kappa light chain. IgG4 isotype antibodies having the hinge-stabilizing mutant S228P are marked with an "x", all others are IgG1 isotype. A horizontal line within a geometric shape indicates an afucosylated antibody. Aglycosylated antibodies obtained via replacement of Asn297 with Ala are indicated by a dot within a geometric shape. Antibodies with Fc amino acid substitutions to modulate FcgammaR binding are indicated by a vertical line within a geometric shape.

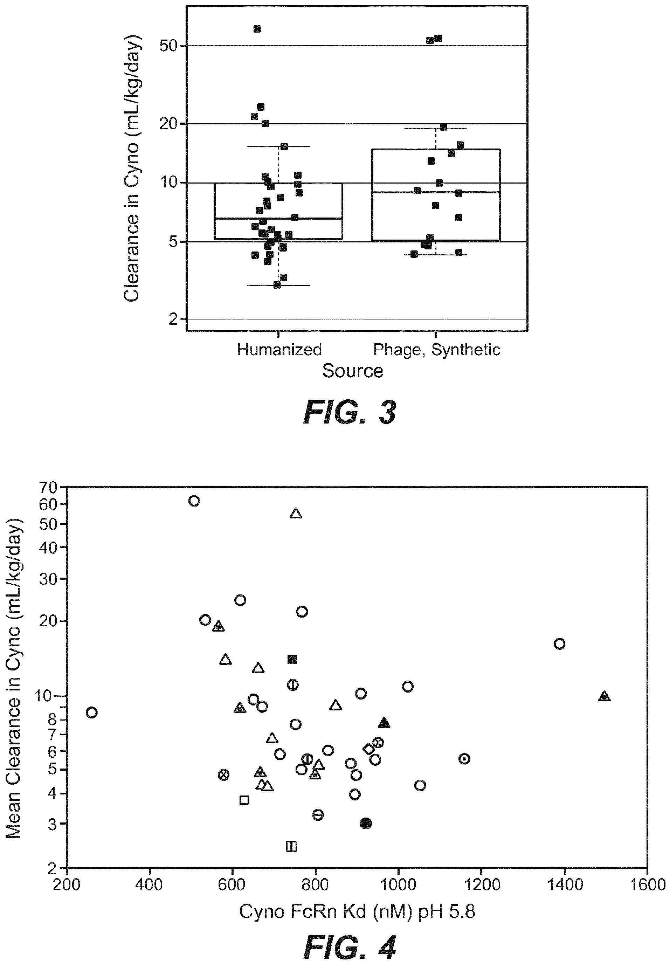

Figure 3 is a boxplot showing the comparison of clearance values in cynomolgus monkeys of humanized and synthetic human antibodies derived from phage display libraries. Chimeric antibodies (n=1) and human antibodies from non-phage sources (n=3) are not shown because of the very small size of the data sets. Plots show individual data values overlaid by boxplots. Boxplot rectangle shows the interquartile range (IQR) between the first quartile (25^{th} percentile) and the third quartile (75^{th} percentile); the thicker horizontal line within the boxplot rectangle is at the median (50^{th} percentile). The lower boxplot whisker extends to the lowest data value that is still within 1.5 IQR of the lower quartile, and the upper whisker extends to the highest data value within 1.5 IQR of the upper quartile.
Figure 4 illustrates the relationship between antibody clearance in cynomolgus monkeys and binding affinity (*K_{D},* pH 5.8) for cynomolgus monkey FcRn. Symbols as defined in Figure 2 are used. Dissociation constants (*K_{D}*) in nM were determined from steady-state analysis of SPR data as described by Yeung *et al.,* 2010. The symbols used are the same as for Figure 2.

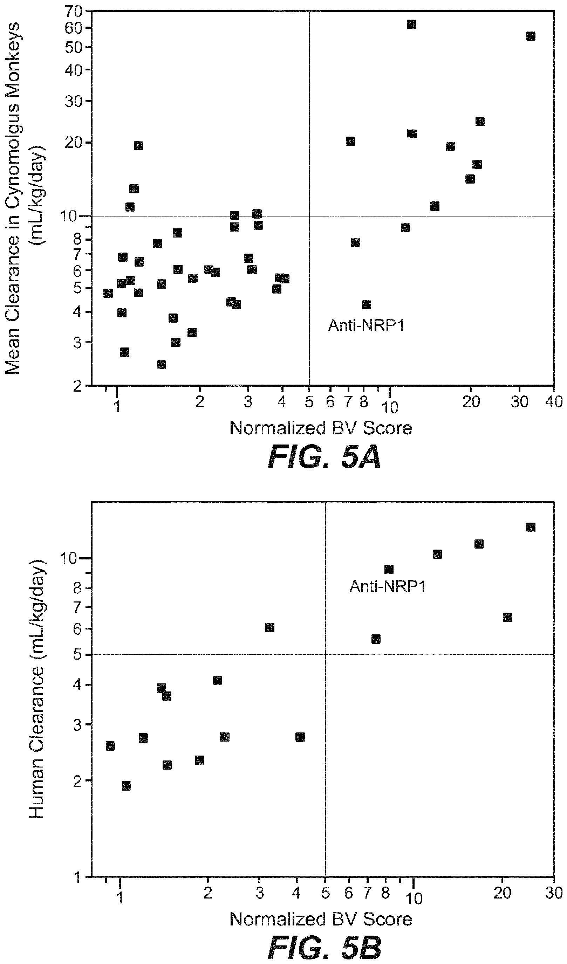

Figure 5 illustrates the association of antibody clearance value in A) cynomolgus monkey and B) human with normalized BV ELISA score. A BV ELISA score > 5 is associated with increased risk of fast clearance in cynomolgus monkey (ρ =0.53, n=45). The BV score was calculated from the mean of 6 determinations; each determination was normalized by dividing by the signal observed for non-coated wells on the same assay plate. Of antibodies with a BV score < 5, 12% have clearance > 10 mL/kg/day in cynomolgous monkey, while clearance exceeds 10 mL/kg/day for 75% of antibodies with BV score > 5. A maximum likelihood estimate for the odds ratio is 19.5 (Fisher's Exact Test, 95% Confidence Interval (3.3, 165.7)). The confidence interval suggests a 3.3 to 166-fold increase in the odds of faster clearance for BV > 5; that the interval does not contain 1 implies statistical significance. B) BV ELISA score > 5 is associated with increased risk of fast clearance in human (p = 0.83, n=16).

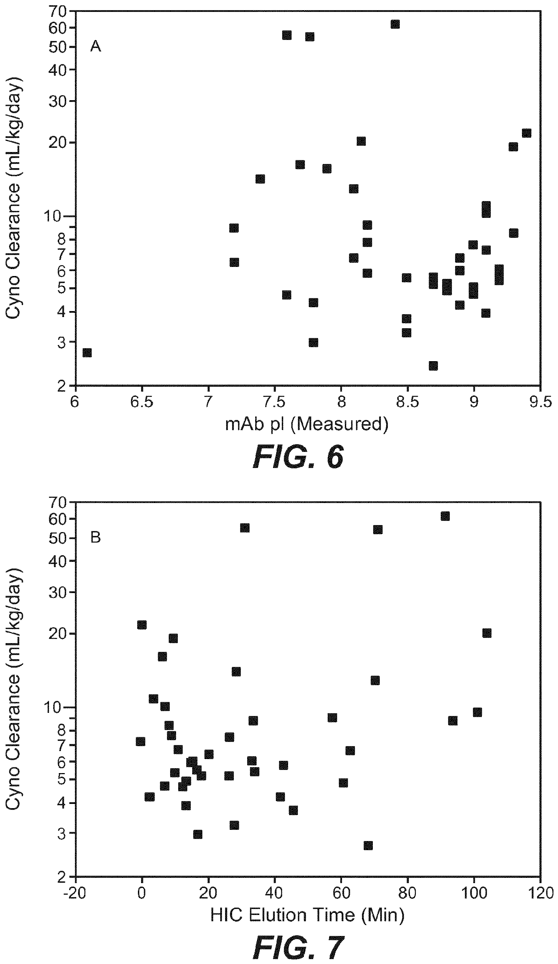

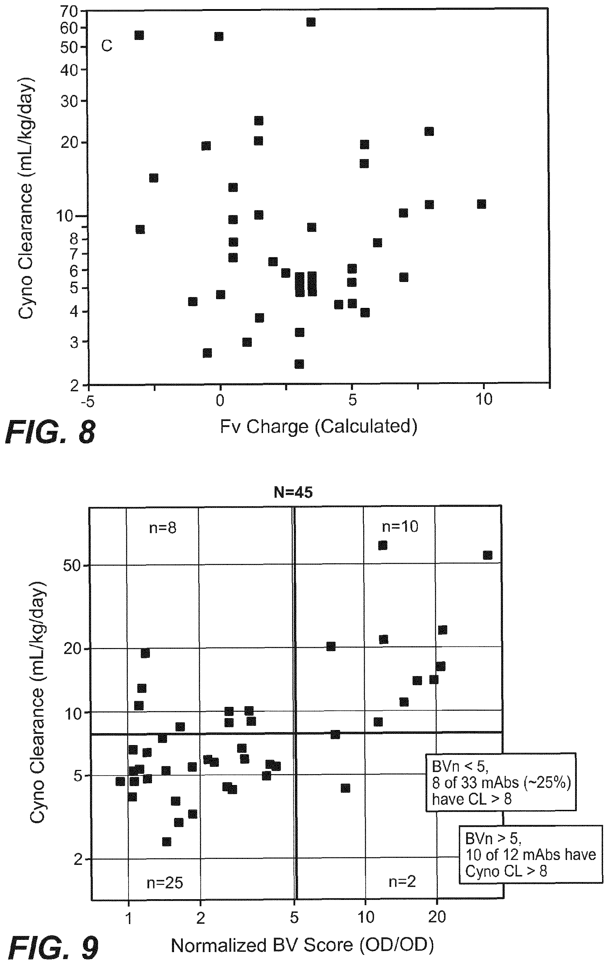

Figures 6-8 show the relationship between antibody clearance in cynomolgus monkeys and antibody with regard to A) pI, B) hydrophobicity as measured by HIC, and C) charge calculated for Fv domain, respectively.
Figure 9 shows that cyno clearance and BV ELISA scores are correlated.

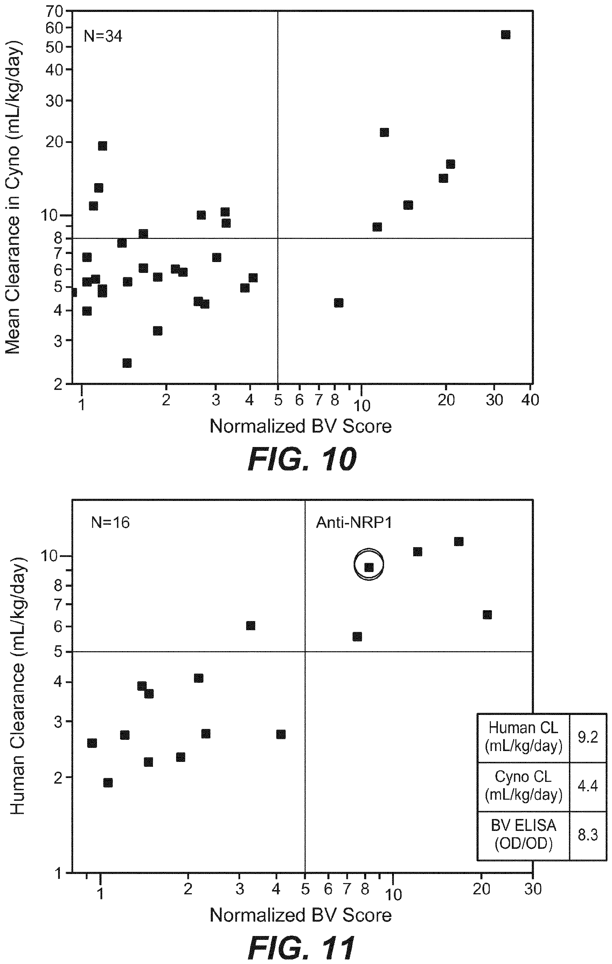

Figure 10 shows that the correlation between cyno clearance and normalized BV score persists between the IgG1 antibodies tested having HIII and kappa1 frameworks and differing CDRs and vernier positions.
Figure 11 shows that the correlation between human clearance and BV ELISA scores persist, even for anti-NRP-1 antibodies.

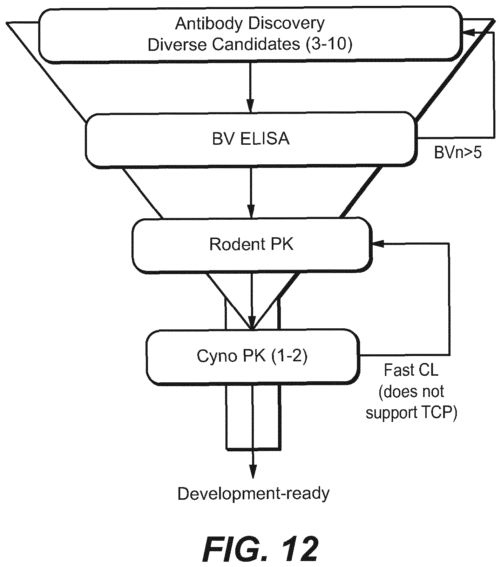

Figure 12 shows a possible screening cascade to mitigate risk of fast clearance.

### DETAILED DESCRIPTION

Here we describe an assay based on detection of binding to baculovirus (BV) particles that is useful to evaluate the off-target binding of therapeutic agents. This assay can be used, among other things, during antibody lead generation or optimization to increase the probability of obtaining a suitable drug.

By testing of a large panel of antibodies in an assay of this invention and analyzing the test results with the same antibodies' pharmacokinetic data in cynomolgus monkeys and, if available, in humans, we determined that the an alteration in the interaction with the recycling FcRn receptor did not account for the faster than expected clearance observed for these antibodies. We did not find that clearance is associated with isoelectric point (pI) or hydrophobicity of the intact antibody as reported by others. We believe that off-target binding accounts for the fast clearance of many antibodies although in most cases such off targets have not been identified. It is time consuming and expensive to conduct pharmacokinetic studies in non-human primates and humans. Prior to this invention, it was hard to predict potential off-target binding contributions a pharmacokinetic profile prior to conducting such pharmacokinetic studies in vivo. We developed an inexpensive, higher throughput, in vitro assay that is simple to employ and can be used to help select therapeutic candidates with greater likelihood of having adequate pharmacokinetic properties and deselect therapeutic candidates with greater likelihood of having inadequate pharmacokinetic properties in non-human primates and humans.

The invention will now be described in detail by way of reference only using the following definitions and examples. All patents and publications, including all sequences disclosed within such patents and publications, referred to herein are expressly incorporated by reference.

"Therapeutic agent" refers to an agent that may be useful in the treatment of a disease. In one embodiment, a therapeutic agent comprises on or more polypeptide sequences. In another embodiment, the therapeutic agent comprises the sequence of an antibody. In another embodiment, the therapeutic agent is an immunoconjugate.

"BV" as used herein refers to a baculovirus particle.

"BV score" as used herein refers to a value calculated from the mean of the values from multiple (2 or more) binding assays measuring the level of binding of a therapeutic agent to a baculovirus particle.

A "normalized BV score" refers to a BV score, wherein the value from each binding assay has been normalized prior to calculating the mean value. In one embodiment, normalization is achieved by dividing the value from each binding assay with BV by a value observed in a non-treated (i.e., without BV) assay. In one example, the OD value from each binding assay with BV is divided by the average value of the OD values observed for non-coated wells.

"Increased risk" or "increased likelihood" refers to the greater possibility that an event will happen.

"Fast clearance" refers to the clearance rate of an agent in a human or cyno. In one embodiment, a fast clearance rate is any rate that is greater than 10 mL/kg/day. In another embodiment, a fast clearance rate is any rate that is greater than 12mL/kg/day.

"Desirable clearance" refers to a desireable clearance rate of an agent in a human or cyno. In one embodiment, a desireable clearance rate is one that is 8.5 mL/kg/day or less. In another embodiment, a desireable clearance rate is one that is 12 mL/kg/day or less.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin can be obtained from any immunoglobulin, such as IgG-1, IgG-2, IgG-3, or IgG-4 subtypes, IgA (including IgA-1 and IgA-2), IgE, IgD or IgM.

A "fusion protein" and a "fusion polypeptide" refer to a polypeptide having at least two portions covalently linked together, where each of the portions is a polypeptide having a different property. The property may be a biological property, such as activity in vitro or in vivo. The property may also be a simple chemical or physical property, such as binding to a target molecule, catalysis of a reaction, etc. The portions may be linked directly by a single peptide bond or through a peptide linker containing one or more amino acid residues. Generally, the portions and the linker will be in reading frame with each other.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "cytotoxic agent" as used herein refers to a substance that inhibits or prevents a cellular function and/or causes cell death or destruction. Cytotoxic agents include, but are not limited to, radioactive isotopes (e.g., At211, 1131, 1125, Y90, Re186, Re188, Sm153, Bi212, P32, Pb212 and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

An "immunoconjugate" is an antibody conjugated to one or more heterologous molecule(s), including but not limited to a cytotoxic agent or a detection agent..

The term "detecting" is intended to include determining the presence or absence of a substance or quantifying the amount of a substance. The term thus refers to the use of the materials, compositions, and methods of the present invention for qualitative and quantitative determinations.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

In a further aspect, an antibody according to any of the above embodiments may incorporate any of the features, singly or in combination, as described in Sections 1-7 below:

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10-8 M or less, e.g. from 10-8 M to 10-13 M, e.g., from 10-9 M to 10-13 M).

In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (125I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [125I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 µl/well of scintillant (MICROSCINT-20 TM; Packard) is added, and the plates are counted on a TOPCOUNT TM gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE®-2000 or a BIACORE ®-3000 (BIAcore, Inc., Piscataway, NJ) at 25°C with immobilized antigen CM5 chips at -10 response units (RU). Briefly, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (-0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25°C at a flow rate of approximately 25 µl/min. Association rates (kon) and dissociation rates (koff) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio koff/kon. See, e.g., Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm bandpass) at 25oC of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO TM spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')2, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. E. coli or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSETM technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for [[PRO]] and the other is for any other antigen. In certain embodiments, bispecific antibodies may bind to two different epitopes of [[PRO]]. Bispecific antibodies may also be used to localize cytotoxic agents to cells which express [[PRO]]. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to [[PRO]] as well as another, different antigen (see, US 2008/0069820, for example).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "conservative substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the invention contemplates an antibody variant that possesses some but not all effector functions, which make it a desirable candidate for applications in which the half life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks Fc R binding (hence likely lacking ADCC activity), but retains FcRn binding ability. The primary cells for mediating ADCC, NK cells, express Fc RIII only, whereas monocytes express Fc RI, Fc RII and Fc RIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).) In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### Recombinant Methods and Compositions

Antibodies may be produced using recombinant methods and compositions, e.g., as described in U.S. Patent No. 4,816,567. In one embodiment, isolated nucleic acid encoding an antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US Patent Nos. 5,959,177, 6,040,498, 6,420,548, 7,125,978, and 6,417,429 (describing PLANTIBODIESTM technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

In the experimental disclosure which follows, the following abbreviations apply: eq (equivalents); M (Molar); µM (micromolar); N (Normal); mol (moles); mmol (millimoles); µmol (micromoles); nmol (nanomoles); g (grams); mg (milligrams); kg (kilograms); µg (micrograms); L (liters); ml (milliliters); µl (microliters); cm (centimeters); mm (millimeters); µm (micrometers); nm (nanometers); ° C. (degrees Centigrade); h (hours); min (minutes); sec (seconds); msec (milliseconds); Ci (Curies) mCi (milliCuries); µCi (microCuries); TLC (thin layer achromatography); Ts (tosyl); Bn (benzyl); Ph (phenyl); Ms (mesyl); Et (ethyl), Me (methyl).

### EXAMPLES

The present invention is described in further detain in the following examples which are not in any way intended to limit the scope of the invention as claimed. The attached Figures are meant to be considered as integral parts of the specification and description of the invention. All references cited are herein specifically incorporated by reference for all that is described therein. The following examples are offered to illustrate, but not to limit the claimed invention.

This application incorporates by reference the patent applications and patents and publications cited herein, in their entirety, including specifically Hotzel et al, (2012), "A Strategy for Risk Mitigation of Antibodies with Fast Clearance", mAbs 4:6, 1-8.

### Example 1

### Determination of clearance in cynomolgus monkeys

This example illustrates the standard method used to determine clearance of an antibody in a non-human primate.

Antibody pharmacokinetics were determined following intravenous administration of a single dose or multiple doses of the therapeutic antibody in cynomolgus monkeys. Serum samples were prepared from blood collected at various time points and antibody concentration was determined by ELISA. In most cases the ELISA consisted of capture with coated antigen followed by detection with anti-human Fc antibody. ELISA assay of antibody 26 employed an anti-idiotype antibody for capture. Antibodies 14, 17, 24, 33, and 44 used an anti-human Fc sandwich ELISA for determination of serum concentration. Serum concentration versus time profiles were analyzed using non-compartmental analysis to calculate the total clearance (Deng, R. et al. Pharmacokinetics of humanized monoclonal anti-tumor necrosis factor-{alpha} antibody and its neonatal Fc receptor variants in mice and cynomolgus monkeys. Drug metabolism and disposition: the biological fate of chemicals 38, 600-605 (2010)). The clearance at the highest dose tested for each antibody was reported as the non-specific clearance, and it was assumed that the contribution of specific clearance to the total clearance was negligible.

Using a slightly larger data set than previously available (Deng, R. et al. Projecting human pharmacokinetics of therapeutic antibodies from nonclinical data: What have we learned? mAbs 3, 61-66 (2011)), we confirm a strong correlation between the clearance values measured in cynomolgus monkeys and humans (Fig. 1, Spearman's correlation coefficient (ρ) =0.74). A simple scaling guideline is obtained in that the clearance measured in humans is about 2-fold slower than the clearance measured in cynomolgus monkeys, with the anti-NRPl antibody as an outlier.

### Example 2

### Determination of FcRn affinity

The following example details how the equilibrium dissociation constants (*K_{D}*) may be determined.

Equilibrium dissociation constants (*K_{D}*) for binding of cynomolgus monkey FcRn to immobilized antibody were determined from surface plasmon resonance (SPR) measurements on a Biacore® 4000 instrument (GE Healthcare). Cynomolgus monkey FcRn was prepared as described previously (Yeung, Y.A. et al. Engineering human IgG1 affinity to human neonatal Fc receptor: impact of affinity improvement on pharmacokinetics in primates. J Immunol 182, 7663-7671 (2009)). A series S sensor chip was docked, normalized and prepared for hydrodynamic addressing using a protocol supplied by the manufacturer. All 5 spots of each of 4 flow cells were activated for amine coupling through 10 minute exposure to EDC/NHS solution (0.2M N-ethyl-N'-(3-diethylaminopropyl)-carbodiimide (EDC) and 0.05M N-hydroxysuccinimide (NHS).. Antibody was coupled to spots 1, 2, 4, and 5 via 7 minute exposure to a solution containing 2 µg/mL antibody and 10 mM NaOAc, pH 5. Unreacted sites were then blocked through 7 minute exposure of all 5 spots to 1 M ethanolamine. In this format spot 3 is the reference cell, 4 different antibodies are coupled per flow cell, with a total of 16 per sensor chip. Coupling densities were in the range of 200-1000 RU of antibody. Separate experiments indicated that calculated *K_{D}* did not vary with coupling density in this range (data not shown). A series of solutions of cynomolgus monkey FcRn varied in concentration from 10 µM to 0.04 µM in 2-fold increments were prepared in a running buffer containing 25 mM MES, 25 mM HEPES, 150 mM NaCl, 0.05% Polysorbate-20, pH 5.8. Sensorgrams were collected for injection of 60 µL of these solutions, and a buffer only control, over the sensor chip at a flow rate of 30 µL/min. The temperature of measurement was 25°C, dissociation was monitored for 180 seconds and then any FcRn remaining bound was eluted by injection of 30 µL of a solution containing 50 mM Tris-HCl, 150 mM NaCl, 0.05% Polysorbate-20, pH 8. Dissociation constants were calculated from Steady State Affinity Analysis using Biacore 4000 Evaluation Software 1.0.

### Example 3

### Production of antigen for ELISA

This example describes the production of the substrate used to coat microtiter plates for use in the assay described herein.

Baculovirus particles were obtained by infecting 1.8 x 10⁹ Sf9 insect cells in 600 ml of serum-free ESF921 media (Expression systems, Davis, CA) with a recombinant *Autographa californica nucleopolyhedrovirus* expressing green fluorescent protein (Bac-to-Bac, Invitrogen) at a multiplicity of infection of about 1. Infected cultures were incubated for 40 hours at 27°C under agitation (200 rpm), harvested and cells removed by centrifugation at 5,000 x g for 10 minutes. The virus in the supernatant was pelleted by centrifugation at 25,000 x g for 4 hours at 4°C, resuspended in PBS buffer (150 mM NaCl, 10 mM sodium phosphate, pH 7.4), layered on a 4 ml 35% (w/v) sucrose cushion in PBS and centrifuged in an SW40Ti rotor (Beckman) at 30,000 rpm for 1 hour at 4°C. The supernatant with debris was discarded, the virus pellet was gently rinsed once with PBS, resuspended in 1.2 ml of PBS with protease inhibitor cocktail (Roche) and stored at 4°C for up to 4 months.

Crude Sf9 cell membranes fractions were obtained from uninfected cells. A total of 2 x 10⁸ uninfected Sf9 cells grown in serum-free ESF media were washed once in PBS and resuspended in 4 ml of ice-cold lysis buffer (1 mM EDTA, 50 mM HEPES buffer, pH 7.4, complete protease inhibitor cocktail). The cells were transferred to a Dounce homogenizer and broken up with 8 strokes of a loose-fitting pestle. An additional 4 ml of lysis buffer containing 0.5 M sucrose were added to the lysed cells and cells were further broken up with 8 strokes of a tight-fitting pestle. The lysate was centrifuged for 10 minutes at 500 x g at 4°C to remove mitochondria, nuclei and coarse debris. The supernatant was transferred to a new tube and centrifuged at 25,000 x g for 20 minutes at 4°C. The pellet was rinsed once with lysis buffer and resuspended in 5 ml of the same buffer using a Dounce homogenizer with a tight-fitting pestle. Large debris were removed by centrifugation at 500 x g at 4°C.

### Example 4

### ELISA assay

Antigen (e.g., baculovirus particles) was immobilized in 384-well ELISA plates (Nunc Maxisorp) by adding 25 µl of a 1% baculovirus suspension in 50 mM sodium carbonate buffer pH 9.6 to each well, allowing the particles to adsorb to the plates overnight at 4°C. The wells were blocked with 50 µl of blocking buffer (PBS containing 0.5% BSA) for 1 hour at room temperature. After rinsing the plates three times with PBS, purified antibodies were serially diluted in blocking buffer, 25 µl were added in duplicate to the ELISA wells and incubated for 1 hour at room temperature. Plates were washed 6 times with PBS and 25 µl of 10ng/ml goat anti-human IgG, (Fcgamma fragment specific) conjugated to horseradish peroxidase (Jackson) were added to each well. The plates were incubated for 1 hour at room temperature, washed 6 times in PBS and 25 µl of TMB substrate added to each well. Reactions were stopped after 15 minutes by adding 25 µl of 1 M phosphoric acid to each well and absorbances were read at 450 nm, referenced at 620nm. BV score was calculated from the mean of 6 optical density determinations each of which had been normalized by dividing by the average signal observed for non-coated wells. For assays with Sf9 cell membranes, a 2% suspension of the crude membranes were used to coat ELISA plates instead of baculovirus particles. Detergents were not added to buffers in any step.

### Example 5

### FACS analysis

HEK293 cells were resuspended in DMEM-10% fetal bovine serum at 5 x 10⁶ cells/ml and dispensed in U-bottom 96-well plates at 100 µl/well. An equal volume of IgG (30 to 50 nM final concentration) diluted in PBS (150 mM NaCl, 10 mM sodium phosphate, pH 7.4) was added to the cells and incubated for 1 h a 4°C. Cells were then washed 3 times with ice-cold PBS, incubated with an anti-human IgG Fc-R-Phycoerythrin conjugate (Jackson Immunoresearch) or an anti-human IgG-Alexa488 (Molecular Probes) conjugate for 30 min at 4°C, washed twice in ice-cold PBS and fixed in PBS with 0.1% paraformaldehyde. Cells were analyzed in a FACSCalibur flow cytometer (BD Biosciences) with a high-throughput sampler.

### Example 6

### Statistical analysis

All statistical analyses were done using R (R Development Core Team (2011). R: A language and environment for statistical computing. R Foundation for Statistical Computing, Vienna, Austria. ISBN 3-900051-07-0, available on-line at R-project[dot]org). The assessment of the performance of the BV score in correctly identifying fast- and slow-clearing antibodies using the odds ratio as described in the legend to Figure 5 is optimistic because it was made using the same dataset used to determine the binning threshold of BV=5. A more conservative assessment of the in vitro assay's utility was obtained for the clearance threshold of 10 mL/kg/day using 5-fold cross-validation and regression modeling to determine the BV score threshold; this approach gave a more modest odds ratio estimate of 14.1 (95% Confidence Interval (2.4,113.3)).

### Example 7

### Predictive scaling of human clearance from clearance in monkeys

Using a slightly larger data set than previously available 8, we confirm a strong correlation between the clearance values measured in cynomolgus monkeys and humans (Fig. 1, Spearman's correlation coefficient (p)=0.74). A simple scaling guideline is obtained in that the clearance measured in humans is about 2-fold slower than the clearance measured in cynomolgus monkeys, with the anti-NRPl antibody as an outlier.

### Example 8

### Characterization of structural variants and clearance in cynomolgus monkeys for a panel of therapeutic antibodies

In the normal course of preclinical development, pharmacokinetic data in cynomolgus monkeys have been collected for 52 humanized and human antibodies. The predominant isotype of these antibodies, is IgG1, kappa (n=46). This antibody panel also includes 4 IgG1, lambda, and 2 IgG4, kappa antibodies. The IgG4 antibodies incorporate the S228P hinge-stabilizing substitution 17. Five of the 52 antibodies are aglycosylated to reduce effector function, 1 is afucosylated to increase ADCC activity, and 3 have Fc amino acid substitutions to ablate or increase FcgammaR binding. The variable domains of these antibodies are either humanized (n=32), derived from synthetic human antibody phage libraries18 (n=16), human (n=3), or mouse (n=1; Rituximab). These antibodies show a wide range of clearance values (Fig. 2) with 15 (29%) antibodies having clearance faster than 10 mL/kg/day.

The hypothesis that fast clearance is associated with the synthetic library antibodies was investigated. Antibodies derived from synthetic human antibody phage libraries were selected and optimized through in vitro selection18. Most of these required affinity maturation through amino acid changes in their complementarity-determining regions (CDRs) in order to achieve the required affinity and potency. Although many of the humanized antibodies also have CDR substitutions relative to the rodent sequence either to improve affinity or chemical stability, in general the humanized antibodies have been subjected to less in vitro optimization. A concern with in vitro selection is that it may inadvertently result in off-target interactions since the negative selection that can be performed in vitro is less extensive than can occur in vivo13. Nonetheless, a comparison of humanized and synthetic library derived antibodies indicates an overlapping range of clearance values (Fig. 3). The median clearance value is 6.5 mL/kg/day for humanized antibodies and 9.0 mL/kg/day for human antibodies from synthetic phage libraries. These data do not provide evidence for an association between the synthetic library antibodies and fast clearance (P = 0.28, Wilcoxon test).

### Example 9

### Binding affinity to cynomolgus monkeys

In order to test if variations in FcRn binding could account for the range of clearance values observed, we used surface plasmon resonance (SPR) methods to determine KD values for binding of these antibodies to purified cynomolgus monkey FcRn at pH 5.8. Since IgG-FcRn binding is a relatively weak interaction, we used a steady-state approach described previously 11 to determine binding constants. Avidity effects arising from 2:1 FcRn:IgG interaction were avoided by immobilizing each of the antibodies (n=44) that were available for analysis. KD values determined in this fashion ranged from about 250 nM to 1,500 nM. Since each antibody was separately immobilized using random coupling via amino groups, with a concomitant uncertainty in the orientation on the sensor chip surface, a range in KD values was not unexpected. Multiple (n=7) determinations of the KD for Pertuzumab indicated a mean of 940±140 (S.D.) nM. The measurement method can detect changes in FcRn affinity as we could reproduce binding effects (data not shown) for Fc mutants known to increase affinity for FcRn 19. The faster clearance observed for some antibodies is not due to an altered low pH binding affinity for cynomolgus monkey FcRn (Fig. 4). Indeed, a comparison of several antibodies with approximately identical FcRn binding affinities gave a nearly 30-fold range in clearance values. Separate experiments (data not shown) indicated that the rate of dissociation of cynomolgus monkey FcRn from these antibodies at pH 7.4 was rapid and equivalent across the panel tested. Although these experiments are insufficient to rule out a change in the recycling pathway for some antibodies as an explanation for the fast clearance observed, it appears unlikely that a significant change in binding affinity at pH 5.8 or in release kinetics at neutral pH play a major role in the fast clearance.

### Example 10

### Development of in vitro assay for risk mitigation of fast clearance

Fast clearance observed for a few antibodies has been associated with specific 14, 15 or non-specific 13, 16 off-target binding. During antibody generation and optimization, most of the antibodies in our panel were screened for lack of binding to closely related antigen homologues. In addition, many of the antibodies derived via phage display were selected in the presence of additives to prevent enrichment for non-specific binding²⁰. As noted by Wu et al. 13, screening by ELISA with a limited variety of antigens may not detect the kind of specific or non-specific off-target binding that may influence in vivo behavior. Therefore, we sought to develop an in vitro assay useful for identifying candidate antibodies likely to show fast clearance. In the course of experiments using intact BV particles bearing specific membrane bound targets as antigen in ELISA assays²¹, we noted that some antibodies bound to baculovirus particles that did not express the target. Many of these antibodies were also shown to bind non-specifically to non-expressing human 293 cells by FACS (data not shown). However, expression of some cognate antigens on 293 cells made it challenging to distinguish specific from non-specific binding.

An initial screen against a test set of 15 antibodies selected from the panel of Figure 2 suggested that fast clearing antibodies bound non-specifically in the baculovirus (BV) ELISA. Subsequently, a larger group of 45 antibodies was tested in the assay. While there is considerable variability, a higher score in the in vitro assay is associated with faster clearance in cynomolgus monkey (Fig. 5). Thresholds of > 10 mL/kg/day for "fast" clearance and > 5 for normalized BV score are useful for quantifying these data. A clearance value of 10 mL/kg/day is more that 1 standard deviation from the mean value (6.5±2.9) determined for 12 human IgG1 antibodies in monkeys8. With these thresholds, 9 antibodies are correctly identified ("true positives") as having fast clearance in monkeys, with only 3 "false positives" - antibodies with clearance values < 10 mL/kg/day that show a normalized BV score > 5. Four antibodies are "false negatives", having clearance greater than 10 mL/kg/day and a BV score < 5. The remaining 29 antibodies are correctly identified as "true negatives".

Although the data set (n=16) is smaller, an increased risk of fast clearance in humans is also associated with high BV score (Fig. 5B; Spearman's ρ = 0.83). Five antibodies with clearance faster than 5 mL/kg/day in humans are correctly identified in the assay. Notably, the anti-NRPl antibody that had clearance < 10 mL/kg/day in cynomolgus monkeys is detected as a true positive for human clearance in the BV ELISA. One antibody having clearance >5 mL/kg/day in humans, and > 10 mL/kg/day in cynomolgus monkeys, is not identified in the assay. The remaining 10 antibodies were correctly classified as having slow clearance in humans.

We tested whether the BV ELISA could be used prospectively to help aid candidate selection through further engineering of antibody 47. This is a humanized antibody with amino acid changes in the CDRs both for affinity improvement and to remove potential sequence liabilities. Cynomolgus monkey is a non-target-binding species for antibody 47. No reproducible tissue staining was observed in monkeys with antibody 47, but a clearance of 20.2 mL/kg/day in cynomolgus monkeys was determined for this antibody. A variant of antibody 47 that retained 2 of the affinity maturation changes, and an amino acid change to remove a potential N-linked glycosylation site in one of the heavy chain CDRs (antibody 47b), showed reduced binding in the BV ELISA (Table 2). Antibody 47c retains only the amino acid change to remove the potential N-linked glycosylation site and also had reduced BV binding. Separate experiments indicated that a large contribution to the BV binding of antibody 47 was made by the VL-D27cS change to remove a potential Asp-Gly isomerization site. Antibodies 47b and 47c had slower clearance relative to antibody 47 in cynomolgus monkeys (Table 2).

### Example 11

The broad distribution of clearance values in cynomolgus monkeys measured for this panel of antibodies, as well as the faster clearance (> 10 mL/kg/day) observed for 15 of the antibodies, was unexpected. Since 40 out of the 52 antibodies have identical human IgG1 Fc sequences, it was expected that faster clearance would not be associated with changes in FcRn interaction. Indeed, although the KD measured for FcRn binding at pH 5.8 varies over a 7-fold range, this is not associated with a trend in clearance values. Given that relatively large increases in pH 5.8 FcRn affinity lead to modest improvement in clearance¹⁹, it is not surprising that the small differences in FcRn affinity determined here do not have an impact on clearance. All of the antibodies tested showed equivalent and rapid release from FcRn at neutral pH. In contrast to the results reported by Wang et al·²², we do not detect an association between clearance and an impact of the Fab portion of the antibody on FcRn release kinetics at neutral pH. Our study used a larger panel of antibodies and a different orientation of the SPR experiments to minimize avidity effects on binding.

Given that the greatest difference between antibodies in our panel is in the variable domains, and in particular in the CDRs, it is likely that differences in the composition of these domains contribute to the variability in clearance behavior. Other groups have observed effects on clearance associated with changes in CDR sequence that were not related to differences in binding affinity to the target antigen 13, 16. We do not find that clearance is associated with isoelectric point (pI) or hydrophobicity of the intact antibody (Suppl. Fig. 1A, B). Igawa et al.²³ have previously shown that clearance of a human IgG1 antibody in cynomolgus monkeys can be reduced through changes in the variable domain that result in a lower pI. This effect was observed at a dose where significant antigen-dependent clearance was operating. The mechanism was presumed to be non-FcRn dependent. For a comparison of a large panel of antibodies, with pharmacokinetic data collected at doses where half-life is FcRn-dependent, clearance rates are not dependent on pI. A caveat to this conclusion is that the majority of antibodies in this analysis are clustered in a relatively narrow range of pI values (7.5-9.5) so perhaps a trend would be observed if a wider range of pI was tested. In addition, we find that clearance is not correlated with total charge calculated for the antibody Fv (Suppl. Fig. 1C). Thus, the physiochemical properties of the antibody that result in fast clearance are not easily recognized from sequence comparisons alone and may require more detailed structure-function analysis.

We show here that a simple assay of non-specific binding can be used to identify antibodies with increased risk of having fast clearance in both humans and cynomolgus monkeys. This assay, based on antibody binding to virus particles prepared from baculovirus-infected insect cells, employs a simple ELISA format and can be applied in high throughput fashion. Since pharmacokinetic data are determined in mammalian species, an assay based on non-specific binding to mammalian cells might have been expected to show a stronger correlation with fast clearance. Preliminary results indicated a correlation between fast clearance and non-specific binding to human 293 cells detected by FACS, but broad application of this assay was precluded because many of the cognate antigens for this collection of antibodies are expressed on 293 cells. The BV ELISA assay we have developed does not suffer from this target-expression limitation. For antibodies that can be evaluated with both assays, a similar trend in non-specific binding is observed (data not shown). Budded baculovirus virions are stable nanoparticles that mimic infected cell surfaces, presenting a complex mixture of phospholipid, carbohydrate, glycoproteins, extracellular matrix and nucleic acids as well as the viral capsid.^{24,25}. The antigen presumably also contains some insect cell debris including protein, membranes and nucleic acids. Such a mixture lacking the specific targets of therapeutic antibodies while retaining the same overall biochemical complexity as human cells and tissues has advantages in a non-specific binding assay since it may detect different kinds of interactions, electrostatic versus hydrophobic for example, in different antibodies.

An important caveat to the association between antibody clearance in cynomolgus monkeys and binding to BV particles is that, other than for antibody 47, it is an observation based on data collected through normal preclinical development, rather than a designed experiment in which BV-particle binding was systematically varied. Clearance values were obtained from separate studies with widely-varying dose levels and using varying PK assay types. However, where sufficient data was available to make an assessment, the association between fast clearance in cynomolgus monkeys and BV binding was found to persist across dose levels, assay types and antibody sources and target types.

The risk of fast clearance can be reduced by using the assay to minimize introduction of non-specific binding during engineering of an antibody. In antibody 47, both charge reduction and increased hydrophobicity were associated with increased non-specific binding to BV particles. Variants with decreased non-specific binding had slower clearance. The BV ELISA is not expected to be a reliable assay of specific off-target effects. For example, the humanized antibody previously shown to have fast clearance in mice due to unintended specific binding to a complement protein¹⁴ has no detectable binding to the BV particles (not shown). In our experience, specific off-target effects are less prevalent than antibodies with broader off-target binding. Moreover, specific off-target effects can usually be identified through mechanistic studies of fast clearance.

The BV particle ELISA detects most but not all antibodies with rapid clearance in cynomolgus monkeys, with few false positives. Therefore, the assay should be considered as a screening tool to reduce the number of therapeutic antibodies that need to be tested in NHP PK experiments and not as an assay that consistently predicts clearance in cynomolgus monkeys. When multiple candidates with similar potencies against a target are available, such that a small but non-zero rate of false positives is acceptable, this assay is a cost effective tool to minimize risk of fast clearance. The BV ELISA assay could be enhanced if the basis for the false negative results could be identified. Relative to membranes from mammalian cells, the phospholipid composition of insect cell membranes is enriched in phosphatidyl inositol, phosphatidyl choline, and phosphatidyl ethanolamine, with lower levels of the acidic phospholipid phosphatidyl serine, cholesterol, glyco- and sphingolipids ²⁴. The higher amounts of neutral phospholipids may account for the false negatives in the assay if the faster clearance of these antibodies is related to binding to acidic phospholipids on mammalian membranes. Two of the four false negative antibodies have a high net positive charge calculated for the Fv domain. It will be interesting to explore whether the phospholipid composition of the BV particles can be manipulated through growth conditions or cell line differences with a concomitant improvement in the veracity of the non-specific binding assay. These studies could further illuminate features to be avoided during antibody engineering to maintain acceptable pharmacokinetic behavior.

### Example 12

### Antibody clearance optimization

In this example we tested whether the BV ELISA could be used prospectively to help aid candidate selection through further engineering.

Antibody 47 is a humanized antibody with amino acid changes in the CDRs both for affinity improvement and to remove potential sequence liabilities. Cynomolgus monkey is a non-target-binding species for antibody 47. No reproducible tissue staining was observed in monkeys with antibody 47, but a clearance of 20.2 mL/kg/day in cynomolgus monkeys was determined for this antibody. A variant of antibody 47 that retained 2 of the affinity maturation changes, and an amino acid change to remove a potential *N*-linked glycosylation site in one of the heavy chain CDRs (antibody 47b), showed reduced binding in the BV ELISA (Table 1). Antibody 47c retains only the amino acid change to remove the potential *N*-linked glycosylation site and also had reduced BV binding. Separate experiments indicated that a large contribution to the BV binding of antibody 47 was made by the change to remove a potential Asp-Gly isomerization site. Antibodies 47b and 47c had slower clearance relative to antibody 47 in cynomolgus monkeys (Table 1).

Thus, this assay may be used as a rapid assessment of clearance and aid in the development of clinically relevant antibodies with lower clearance relative to a parent antibody.

### CITATION LIST

1. Reichert, J.M. Marketed therapeutic antibodies compendium. mAbs 4 (2012).
2. Wang, W., Wang, E.Q. & Balthasar, J.P. Monoclonal antibody pharmacokinetics and pharmacodynamics. Clinical pharmacology and therapeutics 84, 548-558 (2008).
3. Tabrizi, M.A., Tseng, C.M. & Roskos, L.K. Elimination mechanisms of therapeutic monoclonal antibodies. Drug discovery today 11, 81-88 (2006).
4. Mager, D.E. Target-mediated drug disposition and dynamics. Biochemical pharmacology 72, 1-10 (2006).
5. Mould, D.R. & Green, B. Pharmacokinetics and Pharmacodynamics of Monoclonal Antibodies: Concepts and Lessons for Drug Development. Biodrugs 24, 23-39 (2010).
6. Kuo, T.T. & Aveson, V.G. Neonatal Fc receptor and IgG-based therapeutics. mAbs 3, 422-430 (2011).
7. Keizer, R.J., Huitema, A.D.R., Schellens, J.H.M., Beijnen, J.H. Clinical Pharmacokinetics of Therapeutic Monoclonal Antibodies. Clinical Pharmacokinetics 49, 493-507 (2010).
8. Deng, R. et al. Projecting human pharmacokinetics of therapeutic antibodies from nonclinical data: What have we learned? mAbs 3, 61-66 (2011).
9. Dall'Acqua, W.F., Kiener, P.A. & Wu, H. Properties of human IgG1s engineered for enhanced binding to the neonatal Fc receptor (FcRn). The Journal of biological chemistry 281, 23514-23524 (2006).
10. Hinton, P.R. et al. An Engineered Human IgG1 Antibody with Longer Serum Half-Life. J Immunol 176, 346-356 (2006).
11. Yeung, Y.A. et al. Engineering human IgG1 affinity to human neonatal Fc receptor: impact of affinity improvement on pharmacokinetics in primates. J Immunol 182, 7663-7671 (2009).
12. Zalevsky, J. et al. Enhanced antibody half-life improves in vivo activity. Nature biotechnology 28, 157-159 (2010).
13. Wu, H. et al. Development of motavizumab, an ultra-potent antibody for the prevention of respiratory syncytial virus infection in the upper and lower respiratory tract. Journal of molecular biology 368, 652-665 (2007).
14. Bumbaca, D. et al. Highly specific off-target binding identified and eliminated during the humanization of an antibody against FGF receptor 4. mAbs 3, 376-386 (2011).
15. Vugmeyster, Y. et al. Complex pharmacokinetics of a humanized antibody against human amyloid beta peptide, anti-abeta Ab2, in nonclinical species. Pharmaceutical research 28, 1696-1706 (2011).
16. Vugmeyster, Y. et al. In vitro potency, pharmacokinetic profiles and phamacological activity of optimized anti-IL-21R antibodies in a mouse model of lupus. mAbs 2, 335-346 (2010).
17. Angal, S. et al. A single amino acid substitution abolishes the heterogeneity of chimeric mouse/human (IgG4) antibody. Molecular immunology 30, 105-108 (1993).
18. Lee, C.V. et al. High-affinity human antibodies from phage-displayed synthetic Fab libraries with a single framework scaffold. Journal of molecular biology 340, 1073-1093 (2004).
19. Yeung, Y.A. et al. A therapeutic anti-VEGF antibody with increased potency independent of pharmacokinetic half-life. Cancer research 70, 3269-3277 (2010).
20. Dennis, M.S. & Lowman, H.B. in Phage Display, Vol. 266. (eds. T. Clackson & H.B. Lowman) 61-94 (Oxford University Press, Oxford, UK; 2004).
21. Hotzel, I. et al. Efficient production of antibodies against a mammalian integral membrane protein by phage display. Protein engineering, design & selection : PEDS 24, 679-689 (2011).
22. Wang, W. et al. Monoclonal antibodies with identical Fc sequences can bind to FcRn differentially with pharmacokinetic consequences. Drug metabolism and disposition: the biological fate of chemicals 39, 1469-1477 (2011).
23. Igawa, T. et al. Reduced elimination of IgG antibodies by engineering the variable region. Protein engineering, design & selection : PEDS 23, 385-392 (2010).
24. Marheineke, K. Lipid Composition of Spodoptera frugiperda (Sf9) and Trichplusia ni (Tn) insect cells used for baculovirus infection. FEBS Letters 441, 4 (1998).
25. Wang, R. et al. Proteomics of the Autographa californica nucleopolyhedrovirus budded virions. Journal of virology 84, 7233-7242 (2010).
26. Deng, R. et al. Pharmacokinetics of humanized monoclonal anti-tumor necrosis factor-{alpha} antibody and its neonatal Fc receptor variants in mice and cynomolgus monkeys. Drug metabolism and disposition: the biological fate of chemicals 38, 600-605 (2010).
27. Stone, M. Cross-validatory choice and assessment of statistical predictions. Journal of the Royal Statistical Society Ser. B 36, 111-147 (1974).
28. Geisser, S. The predictive sample reuse method with applications I. Amer. Stat. Assoc. 70, 320-328 (1975).

## Claims

1. A method of predicting whether a therapeutic agent will have a desireable clearance rate in a cynomolgus monkey, a human or a cynomolgus monkey and human, comprising the step of contacting the therapeutic agent with a baculovirus particle (BV), measuring the level of binding of therapeutic agent to the BV and calculating a BV score for the therapeutic agent based on the level of binding, wherein a BV score above a predetermined threshold is indicative of an increased likelihood of fast clearance rate, wherein a BV score below a predetermined threshold is indicative of an increased likelihood of a desireable clearance rate.

2. The method according to claim 1, wherein the BV is bound to a microtiter plate prior to binding the therapeutic agent.

3. The method according any one of claims 1-2, wherein the therapeutic agent is labeled with a detection agent.

4. The method according to any one of claims 1-3, further comprising the step of binding a second agent comprising a detection agent to the therapeutic agent.

5. The method according to any one of claims 3 or 4, wherein a signal from the detection reagent is measured.

6. The method according to any one of claims 1-5, wherein the BV score correlating to greater than 10mL/Kg/day clearance rate in a human is considered a fast clearance rate, in particular wherein the predetermined threshold for the BV score is 5.

7. The method according to claim 1, wherein (i) the therapeutic agent comprises a polypeptide, (ii) the first two steps are incorporated into an ELISA assay, or (iii) the first two steps are incorporated into an ELISA assay.

8. A kit for determining whether a therapeutic agent will have a desireable clearance rate, wherein the kit comprises baculovirus particles and an antibody that binds to human antibodies.

9. The kit according to claim 8, wherein (i) the kit further comprises a microtiter plate, or (ii) the antibody that binds to human antibodies binds to the Fc region of human antibodies.

10. The method according to claim 1, wherein (i) the BV is bound to a solid support, or (ii) no detergents are present at any step.

11. The method according to claim 10, wherein the solid support is a microtiter plate, in particular wherein the binding of the therapeutic agent in the absence of a baculovirus particle is measured to derive a value for use in normalization.

12. A kit for determining whether a therapeutic agent will have a desireable clearance rate, wherein the kit comprises a baculovirus particle and instruction in an ELISA assay.

13. A method of making a therapeutic agent comprising the step of selecting a therapeutic agent that has a desireable clearance rate in humans and expressing the therapeutic agent in a host cell.

14. The method further comprises the step of recovering the therapeutic agent expressed by the host cell.

15. The method of claim 13 or claim 14, wherein selection of a therapeutic agent is achieved by the method of claim 1.
